# EUROPEAN PATENT SPECIFICATION

(11) **EP 1 927 316 B1**
(45) Date of publication and mention of the grant of the patent: **17.10.2012**
(21) Application number: 06125183.1
(22) Date of filing: 01.12.2006
(51) Int. Cl.: A61B 5/00, A61M 25/00

(54) **Sensor and guide wire assembly**
Sensor und Führungsdrahtanordnung
Ensemble de capteur et de fil guide

(43) Date of publication of application: 04.06.2008
(73) Proprietor: RADI MEDICAL SYSTEMS AB, 754 50 Uppsala (SE)
(72) Inventor: Smith, Leif, 756 52, UPPSALA (SE)
(74) Representative: Holmberg, Nils Anders Patrik

(56) References cited:
- EP-A1- 1 479 407
- US-A- 5 226 423
- US-A1- 2002 013 527
- US-A1- 2003 028 128
- US-A1- 2004 225 232

## Description

### Field of the Invention

The invention relates generally to a sensor mounted in a distal portion of a guide wire for intravascular measurements of physiological variables in a living body, and in particular to the design of said distal portion.

### Background of the invention

Sensor and guide wire assemblies in which a sensor, adapted for measurements of physiological variables in a living body, such as blood pressure and temperature, is mounted at a distal portion of a guide wire are known.

For example, the U.S. Patent No. Re. 35,648, which is assigned to the present assignee, discloses a sensor and guide wire assembly comprising a sensor element, an electronic unit, signal transmitting cables connecting the sensor element to the electronic unit, a flexible tube having the signal cables and the sensor element disposed therein, a solid metal wire, and a coil attached to the distal end of the solid wire. The sensor element comprises a pressure sensitive device, e.g. a membrane, with piezoresistive elements electrically connected in a Wheatstone bridge-type of circuit arrangement mounted thereon.

The sensor element is usually provided in the form of a silicon chip, which besides the pressure sensitive device also comprises integrated electronic circuits. For various reasons it is desired to include ever more functionality in the electronic circuits. This need has so far been met by the general miniaturization trend that exists in the chip technology, which allows more functionality and more complex circuits to be integrated in a comparatively smaller area, or, in the three-dimensional case, in a smaller volume. Such miniaturization is extremely important within the field of guide wire mounted sensors, because the outer diameter of the sensor guide wire is in practice limited by the inner diameter of a catheter which in a so-called PCI (Percutan Coronar Intervention) is used for the treatment of a stenosis present in a blood vessel and which is threaded over the sensor guide wire and advanced to the site of interest. The standard inner diameter of such catheters is 0.35 mm (0.014 inches); and consequently the standard outer diameter of most guide wires, and in particular of sensor guide wires, is therefore 0.35 mm (0.014 inches).

However, the miniaturization of electronic circuits and chips is a difficult task, and it is far from certain that the chips industry can provide sensor chips which fulfil the future requirements for mounting in a guide wire adapted for intravascular measurements.

Consequently, there is a need for a guide wire design that provides more space for a sensor element, while at the same time allows use together with a standard catheter.

### Summary of the Invention

The above-mentioned object is achieved by the present invention according to the independent claims.

Preferred embodiments are set forth in the dependent claims.

Embodiments of the present invention are directed to a sensor and guide wire assembly comprising a sensor element which is mounted in a distal portion of a guide wire. According to embodiments of the invention, a distal portion is provided which has a larger diameter than the rest of the guide wire, to thereby provide more space for the sensor element. The inventor has observed that a catheter very rarely (or never) is advanced over the distal tip of the guide wire, and has realized that a distal portion of a guide wire may have a larger diameter than the rest of the guide wire. A hollow distal portion having a larger diameter provides more space for a sensor element (and/or other devices) mounted within this distal portion.

According to the different embodiments disclosed below, an enlarged distal sensor portion may include the entire distal end of the sensor guide wire, but may also be restricted to the sensor portion, with the short distal end portion of the guide wire being smaller than the enlarged sensor portion.

Further, the sensor and guide wire assembly can be of a relatively well-known design (see, e.g., the above-referenced Re 35,648), with electrical leads that extend along the length of the guide wire and connect the sensor element to a male connector at the proximal end of the sensor guide wire; or the sensor and guide wire assembly can be of a wireless type, in which the sensor element wirelessly communicates with an external unit. Especially in the latter case, there is a need for very complex electronics and therefore a need for more space within the guide wire.

### Brief Description of the Drawings

Fig. 1 is a schematic illustration of a sensor and guide wire assembly according to the prior art.
Fig. 2 shows a cross-sectional view of a first embodiment of a sensor and guide wire assembly according to the present invention.
Fig. 3 shows a cross-sectional view of a second embodiment of a sensor and guide wire assembly according to the present invention.
Fig. 4 shows a cross-sectional view of a third embodiment of a sensor and guide wire assembly according to the present invention.
Fig. 5 shows a cross-sectional view of a fourth embodiment of a sensor and guide wire assembly according to the present invention.

### Detailed Description of Preferred Embodiments

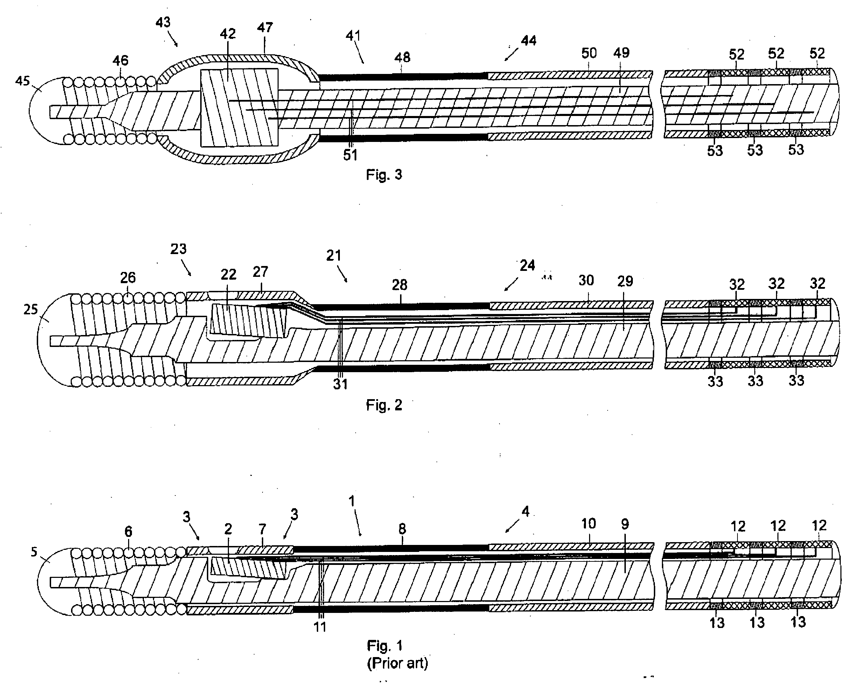

Fig. 1 illustrates schematically the general design of a sensor and guide wire assembly 1 according to the prior art. The sensor and guide wire assembly 1 comprises a sensor element 2, which is arranged in a distal portion 3 of a sensor guide wire 4. More specifically, the sensor guide wire 4 comprises a distal tip 5, a distal coil spring 6, a jacket or sleeve 7, a flexible distal tube 8, which also could be in the form of a coil spring, a core wire 9, and a proximal tube 10. The distal coil spring 6 is attached to the distal tip 5, and extends to the jacket 7, which serves as a housing for the sensor element 2. The flexible distal tube 8 extends between the jacket 7 and the proximal tube 10. The sensor element 2 is mounted in a recess in a distal portion of the core wire 9, and is through a window in the jacket 7 in fluid communication with the medium, e.g. blood, surrounding the sensor and guide wire assembly 1. The sensor and guide wire assembly 1 comprises further a number of signal transmitting cables 11, the distal ends of which are electrically connected to the sensor element 2 and which extend along the core wire 9 to the proximal end portion of the sensor guide wire 4, where each signal transmitting cable 11 is electrically connected to a conductive member 12. The conductive members 12 are electrically insulated from the core wire 9 as well as from each other by insulating members 13, so as to form a male connector adapted for connection to a corresponding female connector of an external signal conditioning and display unit (not shown in Fig. 1) for displaying the measured quantities, e.g. pressure, temperature and/or flow.

From Fig. 1, which is intended to present the essential elements of a sensor and guide wire assembly according to prior art, it may be appreciated that there is only a limited amount of space available for the sensor element. Throughout the present specification, the term "sensor element" as applied to pressure measurement is meant to encompass both the pressure sensitive member, e.g. a membrane, as well as the electronic components and circuits that are connected to the pressure sensitive member. It is even contemplated that a sensor element comprises a pressure sensitive part and an electronic part, which are connected by at least one electrical lead. The latter arrangement is disclosed in the U.S. Patent Applications Nos. 11/063,744 and 11/359,761, both of which are assigned to the present assignee. In a preferred embodiment of these patent applications, the pressure sensitive member and the electronic circuitry are incorporated in a silicon chip with integrated circuits.

A first embodiment of a sensor and guide wire assembly 21 according to the present invention is disclosed in Fig. 2, which illustrates that the sensor and guide wire assembly 21 comprises a sensor element 22, which is arranged in a distal portion 23 of a sensor guide wire 24. More specifically, the sensor guide wire 24 comprises a distal tip 25, a distal coil spring 26, a jacket or sleeve 27, a flexible distal tube 28, a core wire 29, and a proximal tube 30. The distal coil spring 26 is attached to the distal tip 25, and extends to the jacket 27, which serves as a housing for the sensor element 22. The flexible distal tube 28, which also could be in the form of a coil spring, extends between the jacket 27 and the proximal tube 30. The sensor element 22 is mounted in a recess in a distal portion of the core wire 29, and is through a window in the jacket 27 in fluid communication with the medium, e.g. blood, surrounding the sensor and guide wire assembly 21. The sensor and guide wire assembly 21 comprises further a number of signal transmitting cables 31, the distal ends of which are electrically connected to the sensor element 22 and which extend along the core wire 29 to the proximal end portion of the sensor guide wire 24, where each signal transmitting cable 31 is electrically connected to a conductive member 32. The conductive members 32 are electrically insulated from the core wire 29 as well as from each other by insulating members 33, so as to form a male connector adapted for connection to a corresponding female connector of an external signal conditioning and display unit (not shown in Fig. 2).

A comparison between Fig. 1 and Fig. 2 reveals that the sensor guide wire 24 shown in Fig. 2 is provided with a jacket 27 which is enlarged in comparison with the corresponding jacket 7 of the sensor guide wire 4 illustrated in Fig. 1. The enlargement of the jacket 27 provides more space for the sensor element 22 (and/or other components or devices), which consequently can comprise more electrical components and more complex functionality. In the first embodiment illustrated in Fig. 2, the jacket 27 is not the only portion of the sensor guide wire 24 that has a diameter which is enlarged in comparison with the diameters of the more proximal portions of the sensor guide wire 24. As can be seen, the diameters of the distal tip 25 and the distal coil spring 26 are also larger than the diameters of the flexible distal tube 28 and the proximal tube 30. In other words, in this embodiment, an enlarged distal sensor portion 23 encompasses the distal tip 25, the distal coil spring 26 and the jacket 27, and constitutes the extreme distal portion of the sensor guide wire 24. By making the diameter of the distal coil spring 26 equal to the diameter of the jacket 27 the assembly of the sensor and guide wire assembly 21 is simplified, because it is relatively easy to fit the distal coil spring 26 to the jacket 27 and to match the mechanical characteristics of these two elements to each other as there is no abrupt transition from the jacket 27 to the distal coil spring 26.

Fig. 3 illustrates a second, alternative embodiment of a sensor and guide wire assembly 41 according to the present invention, and shows that the sensor and guide wire assembly 41 comprises a sensor element 42, which is arranged in a distal portion 43 of a sensor guide wire 44. More specifically, the sensor guide wire 44 comprises a distal tip 45, a distal coil spring 46, a jacket or sleeve 47, a flexible distal tube 48, a core wire 49, and a proximal tube 50. The distal coil spring 46 is attached to the distal tip 45, and extends to the jacket 47, which serves as a housing for the sensor element 42. The flexible distal tube 48, which also could be in the form of a coil spring, extends between the jacket 47 and the proximal tube 50. The sensor element 42 is mounted at a distal portion of the core wire 49, and is through a window (not visible in Fig. 3) in the jacket 47 in fluid communication with the medium, e.g. blood, surrounding the sensor and guide wire assembly 41. The sensor and guide wire assembly 41 comprises further a number of signal transmitting cables 51, the distal ends of which are electrically connected to the sensor element 42 and which extend along the core wire 49 to the proximal end portion of the sensor guide wire 44, where each signal transmitting cable 51 is electrically connected to a conductive member 52. The conductive members 52 are electrically insulated from the core wire 49 as well as from each other by insulating members 53, so as to form a male connector adapted for connection to a corresponding female connector of an external signal conditioning and display unit (not shown in Fig. 3).

In contrast to the first embodiment depicted in Fig. 2, the jacket 47 constitutes the only portion of the sensor guide wire 44 that has an enlarged diameter; and in particular the distal tip 45 and the distal coil spring 46 have both a diameter that is equal to the diameter of the flexible distal tube 48 and the proximal tube 50. Due to its small diameter, the distal end portion (i.e. the coil spring 46) of the sensor guide wire 44 can be made very flexible, which can be advantageous in some medical applications, such as when the sensor guide wire is manoeuvred through extremely tortuous vessels.

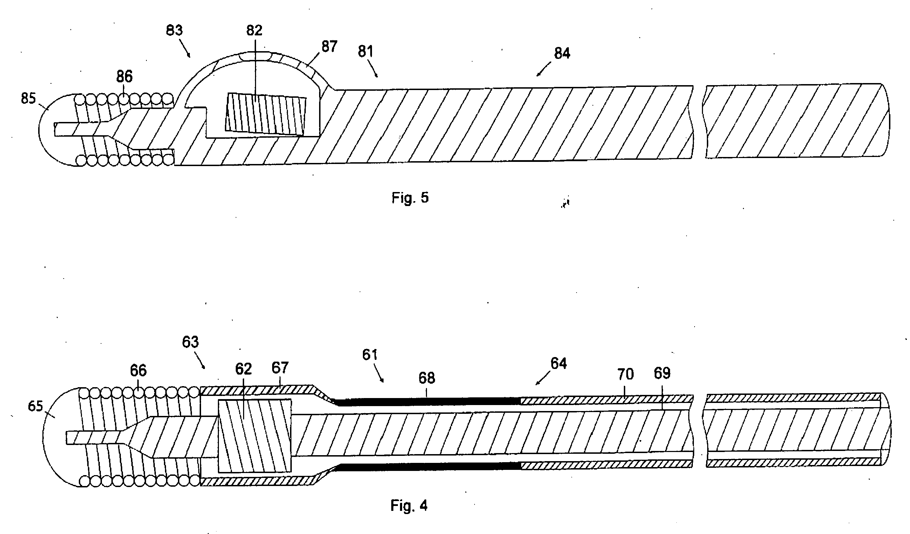

As was briefly mentioned above, it is foreseen that a sensor element can communicate wirelessly with an external unit. In this case, the physical requirements put on the sensor element are considerably higher than in the wired case, because the sensor element must be able to receive energy via electrical or electromagnetic signals and communicate signals that are modulated in accordance with the sensed measurements. A schematic illustration of a wireless sensor and guide wire arrangement is shown in Fig. 4. In this third embodiment of the present invention, a sensor and guide wire assembly 61 comprises a sensor element 62, which is arranged in a distal portion 63 of a sensor guide wire 64.

More specifically, the sensor guide wire 64 comprises a distal tip 65, a distal coil spring 66, a jacket or sleeve 67, a flexible distal tube 68, a core wire 69, and a proximal tube 70. The distal coil spring 66 is attached to the distal tip 65, and extends to the jacket 67, which serves as a housing for the sensor element 62. The flexible distal tube 68, which also could be in the form of a coil spring, extends between the jacket 67 and the proximal tube 70. The sensor element 62 is mounted to a distal portion of the core wire 69, and is through a window (not visible in Fig. 4) in the jacket 67 in fluid communication with the medium, e.g. blood, surrounding the sensor and guide wire assembly 61.

The third embodiment discussed in conjunction with Fig. 4 differs from the first and second embodiments of the present invention shown in Fig. 2 and Fig. 3, respectively, in that there are no signal transmitting cables provided. The sensor element 62 is instead capable of communicating with an external unit by electromagnetic signals, i.e. there is a wireless communication between the sensor element 62 and the external unit (not shown in Fig. 4). In this case, the physical requirements put on a sensor element are considerably higher than in the wired case, since the sensor element must be able to receive energy from some type of carrier signals and then communicate signals that are modulated in accordance with the sensed measurement. Other types of wireless communications are also possible, e.g. that the sensor is powered by a battery and only output signals are received by an external unit. Generally, the more functionality that is incorporated in a sensor element, the larger is the space required for the electronic circuits; and a sensor guide wire that provides a larger space for the sensor element is particularly advantageous for a wireless sensor and guide wire assembly.

In the third embodiment of Fig. 4, the general structure of the sensor guide wire 64 is the same as in the first and second embodiments, i.e. the sensor guide wire 64, like sensor guide wires 24 and 44, respectively, comprises a core wire as well as proximal and distal tubes. When a wireless sensor element is used, the design of a sensor guide wire can be significantly changed, due to the fact that signal transmitting cables can be dispensed with. In a fourth embodiment of the invention, which is schematically illustrated in Fig. 5, a sensor and guide wire assembly 81 adapted for wireless communication comprises a sensor element 82, which is arranged in a distal portion 83 of a sensor guide wire 84. More specifically, the sensor guide wire 84 comprises a distal tip 85, a distal coil spring 86, and a housing 87. The distal coil spring 86 is attached to the distal tip 85, and extends to the housing 87, in which the sensor element 82 is disposed. In this embodiment, it should be noted that there is no separate core wire and no proximal tube, as the sensor guide wire 84 is made in from a solid wire, typically a metal wire. Further, the housing 87 is formed integrally with the solid wire that constitutes the sensor guide wire 84, and it can furthermore be noted that the housing 87 only projects out from a part of the circumference of the sensor guide wire 84. With this design, the mechanical characteristics of the sensor guide wire 84 can be improved, e.g. regarding torsional rigidity and bending stiffness.

As already has been discussed, the present invention is based on the observation that a catheter which is threaded onto a sensor guide wire never is advanced over the distal end of the sensor guide wire, and an enlarged distal sensor portion will consequently not prevent an effective use of a combination of a sensor guide wire and a catheter. As the person skilled in the art will recognize, there is a significant difference between a (sensor) guide wire and a catheter, for example, in that a catheter completely lacks steering capability, i.e. the catheter must be guided to the site of interest by means of a guide wire. For a standard sensor guide wire having an outer diameter of 0.35 mm (0.014 inches), which corresponds to the inner diameter of a standard catheter, the enlarged sensor portion is preferably less than 2 mm, and in particular less than 1 mm. The enlarged portion may be, for example, 25% larger (or 50%, or 100%, or 200%, or 300% larger) than the non-enlarged portion.

Although the present invention has been described with reference to specific embodiments, also shown in the appended drawings, it will be apparent for those skilled in the art that many variations and modifications can be done within the scope of the invention as described in the specification and defined with reference to the claims below. It should in particular be noted that a sensor portion that is enlarged in comparison with the more proximal portions of a sensor guide wire can be symmetrical, i.e. having a larger diameter than the diameters of the more proximal portions but with the same centre, or can be asymmetric or non-circular, i.e. where only a sector of the cross-section has a larger radius than the more proximal portions, an example of which was illustrated in conjunction with Fig. 5, the important feature being that the sensor portion exhibits a maximal cross-sectional dimension that is larger than the maximal cross-sectional dimension of the guide wire portions located proximally of the sensor portion. Said cross-sectional dimension should be taken perpendicular to the central axis of the sensor guide wire. Consequently, the corresponding relationship and definition applies also for a distal end portion being smaller than the enlarged sensor portion, an example of which was discussed in conjunction with Fig. 3.

## Claims

1. Sensor and guide wire assembly (21; 41; 61; 81) for intravascular measurement of a physiological variable in a living body comprising a sensor element (22; 42; 62) mounted at a distal sensor portion (23; 43; 63) of a guide wire (24; 44; 64), that comprises a jacket (27; 47; 67), which serves as a housing for the sensor element (22; 42; 62), **characterized in that** said sensor portion exhibits an enlarged cross-section to accommodate said sensor element, and that said sensor portion has a maximal cross-sectional dimension which is at least 10% larger than a maximal cross-sectional dimension of each of the guide wire portions located proximally of said sensor portion.

2. Sensor and guide wire assembly according to claim 1, **characterized in that** a guide wire portion located distally of said sensor portion exhibits a maximal cross-sectional dimension which is smaller than said maximal cross-sectional dimension of said sensor portion.

3. Sensor and guide wire assembly according to claim 1, **characterized in that** a guide wire portion located distally of said sensor portion exhibits a maximal cross-sectional dimension which is essentially equal to said maximal cross-sectional dimension of said sensor portion.

4. Sensor and guide wire assembly according to any preceding claim, **characterized in that** said sensor portion has a circular cross-section.

5. Sensor and guide wire assembly according to any preceding claim, **characterized in that** said sensor portion has a non-circular cross-section.

6. Sensor and guide wire assembly according to any preceding claim, **characterized in that** said maximal cross-sectional dimension of said guide wire portions located proximally of said sensor portion is 0.35 mm (0.014 inches).

7. Sensor and guide wire assembly according to any preceding claim, **characterized in that** said maximal cross-sectional dimension of said sensor portion is less than 2.0 mm.

8. Sensor and guide wire assembly according to any preceding claim, **characterized in that** said sensor element comprises circuitry for wireless communication of said measurement.

## Patentansprüche

1. Sensor- und Führungsdrahtanordnung (21; 41; 61; 81) zur intravaskulären Messung einer physiologischen Variablen in einem lebenden Körper, wobei die Anordnung ein Sensorelement (22; 42; 62) aufweist, das an einem distalen Sensorabschnitt (23; 43; 63) eines Führungsdrahts (24; 44; 64) montiert ist, der einen Mantel (27; 47; 67) aufweist, der als Gehäuse für das Sensorelement (22; 42; 62) dient, **dadurch gekennzeichnet, dass** der Sensorabschnitt einen vergrößerten Querschnitt aufweist, um das Sensorelement unterzubringen, und dass der Sensorabschnitt eine größte Querschnittabmessung aufweist, die um mindestens 10% größer ist als eine größte Querschnittsabmessung jedes der proximal zu dem Sensorabschnitt angeordneten Führungsdrahtabschnitte.

2. Sensor- und Führungsdrahtanordnung nach Anspruch 1, **dadurch gekennzeichnet, dass** ein distal zu dem Sensorabschnitt angeordneter Führungsdrahtabschnitt eine größte Querschnittabmessung aufweist, die kleiner ist als die größte Querschnittabmessung des Sensorabschnitts.

3. Sensor- und Führungsdrahtanordnung nach Anspruch 1, **dadurch gekennzeichnet, dass** ein distal zu dem Sensorabschnitt angeordneter Führungsdrahtabschnitt eine größte Querschnittabmessung aufweist, die im Wesentlichen gleich der größten Querschnittabmessung des Sensorabschnitts ist.

4. Sensor- und Führungsdrahtanordnung nach einem der vorstehenden Ansprüche, **dadurch gekennzeichnet, dass** der Sensorabschnitt einen runden Querschnitt aufweist.

5. Sensor- und Führungsdrahtanordnung nach einem der vorstehenden Ansprüche, **dadurch gekennzeichnet, dass** der Sensorabschnitt einen nicht runden Querschnitt aufweist.

6. Sensor- und Führungsdrahtanordnung nach einem der vorstehenden Ansprüche, **dadurch gekennzeichnet, dass** die größte Querschnittabmessung der proximal zu dem Sensorabschnitt angeordneten Führungsdrahtabschnitte 0,35 mm (0,014 Zoll) beträgt.

7. Sensor- und Führungsdrahtanordnung nach einem der vorstehenden Ansprüche, **dadurch gekennzeichnet, dass** die größte Querschnittabmessung des Sensorabschnitts kleiner als 2,0 mm ist.

8. Sensor- und Führungsdrahtanordnung nach einem der vorstehenden Ansprüche, **dadurch gekennzeichnet, dass** das Sensorelement Schaltungen zur drahtlosen Übertragung der Messung aufweist.

## Revendications

1. Ensemble de capteur et de fil guide (21 ; 41 ; 61 ; 81) pour la mesure intravasculaire d'une variable physiologique dans un corps vivant, comprenant un élément capteur (22 ; 42 ; 62) monté sur une partie capteur distale (23 ; 43 ; 63) d'un fil guide (24 ; 44 ; 64), qui comprend une enveloppe (27 ; 47 ; 67), qui sert de logement pour l'élément capteur (22 ; 42 ; 62), **caractérisé en ce que** ladite partie capteur présente une section transversale agrandie pour recevoir ledit élément capteur, et **en ce que** ladite partie capteur présente une dimension de section transversale maximale qui est au moins 10 % supérieure à une dimension de section transversale maximale de chacune des parties du fil guide situées de manière proximale par rapport à ladite partie capteur.

2. Ensemble de capteur et de fil guide selon la revendication 1, **caractérisé en ce qu'**une partie du fil guide située de manière distale par rapport à ladite partie capteur présente une dimension de section transversale maximale qui est inférieure à ladite dimension de section transversale maximale de ladite partie capteur.

3. Ensemble de capteur et de fil guide selon la revendication 1, **caractérisé en ce qu'**une partie du fil guide située de manière distale par rapport à ladite partie capteur présente une dimension de section transversale maximale qui est sensiblement égale à ladite dimension de section transversale maximale de ladite partie capteur.

4. Ensemble de capteur et de fil guide selon l'une quelconque des revendications précédentes, **caractérisé en ce que** ladite partie capteur comporte une section transversale circulaire.

5. Ensemble de capteur et de fil guide selon l'une quelconque des revendications précédentes, **caractérisé en ce que** ladite partie capteur comprend une section transversale non circulaire.

6. Ensemble de capteur et de fil guide selon l'une quelconque des revendications précédentes, **caractérisé en ce que** ladite dimension de section transversale maximale desdites parties du fil guide situées de manière proximale par rapport à ladite partie capteur est de 0,35 mm (0,014 pouce).

7. Ensemble de capteur et de fil guide selon l'une quelconque des revendications précédentes, **caractérisé en ce que** ladite dimension de section transversale maximale de ladite partie capteur est inférieure à 2,0 mm.

8. Ensemble de capteur et de fil guide selon l'une quelconque des revendications précédentes, **caractérisé en ce que** ledit élément capteur comprend un circuit pour une communication sans fil de ladite mesure.
